# EUROPÄISCHE PATENTSCHRIFT

(11) **EP 0 640 059 B1**
(45) Veröffentlichungstag und Bekanntmachung des Hinweises auf die Patenterteilung: **08.10.1997**
(21) Anmeldenummer: 93911480.7
(22) Anmeldetag: 20.04.1993
(51) Int. Cl.: C02F 3/28, C12M 1/107

(54) **BIOGASREAKTOR**
BIOGAS REACTOR
REACTEUR A BIOGAZ

(30) Priorität: 21.04.1992 DE 4213015
(43) Veröffentlichungstag der Anmeldung: 01.03.1995
(73) Patentinhaber: Märkl, Herbert, Prof. Dr.-Ing., D-21218 Seevetal (DE)
(72) Erfinder: Märkl, Herbert, Prof. Dr.-Ing., D-21218 Seevetal (DE)
(74) Vertreter: Schmidt-Bogatzky, Jürgen, Dr. Ing.
(86) Internationale Anmeldenummer: EP9300948
(87) Internationale Veröffentlichungsnummer: WO9322240

(56) Entgegenhaltungen:
- EP-A- 0 193 999
- EP-A- 0 311 215
- GB-A- 2 021 550
- US-A- 4 762 612

## Beschreibung

Die Erfindung betrifft einen Biogasreaktor, dessen Reaktorgehäuse ganz oder teilweise mit aktiver Biomasse gefüllt ist, in dem mittels eines oder mehrerer säulenartig übereinander angeordneter Trennelemente mit jeweils einer Überströmkante für aufsteigendes Gas in deren Reaktorabschnitten jeweils ein Gassammelraum zum Sammeln der aus der Gärsuspension aufsteigenden Gase und ein gasblasenarmer Sedimentationsraum zur Sedimentation aktiver Biomasse-Schlammteilchen ausgebildet ist, in den eine Leitung mit einem als Ventil ausgebildeten Drosselorgan zur Gasabführung geführt ist.

In Biogasreaktoren werden Substanzen durch die Einwirkung von Mikroorganismen in CO₂ und Methan als Biogas umgesetzt. Die vorliegende Erfindung betrifft speziell Bioreaktoren, bei denen die beteiligten Mikroorganismen in flüssigem Medium frei suspendiert vorliegen oder auf kleinen Feststoffpartikeln immobilisiert sind. Voraussetzung für eine Anreicherung der Mikroorganismen innerhalb des Reaktors ist in beiden Fällen, daß diese in einer Form existieren, die die Sedimentation innerhalb der Gärsuspension zuläßt. Im ersten Fall ist dies durch eine mehr oder weniger starke Bildung von sedimentationsfähigen, mikrobiellen Agglomeraten gegeben, im zweiten Fall wird ein gegenüber Wasser höheres spezifisches Gewicht durch den Feststoff erreicht, der als Aufwuchsfläche dient, beispielsweise Sand mit einem Partikeldurchmesser kleiner 0,5mm.

Bei hochbauenden Reaktoren, die aufgrund ihres geringen Bedarfs an Grundfläche technisch anzustreben sind, akkumuliert mit zunehmender Höhe das aufsteigende Biogas. Infolge des dabei entstehenden hohen Gasvolumenstroms wird durch Flotation aktive Biomasse in die oberen Reaktorzonen transportiert und aus dem Reaktor ausgetragen. Um die Abscheideleistung des Reaktors zu erhalten, ist es deshalb notwendig, den Gasvolumenstrom zu begrenzen und Biogas schon in tieferen Reaktorzonen zu entnehmen. Gleichzeitig ist die Strömung so zu führen, daß in den verschiedenen Reaktorhöhen Sedimentationszonen entstehen, andererseits aber eine gewisse Durchmischung des Reaktorinhalts gewährleistet ist. Da eine geeignete Balance zwischen diesen an sich einander entgegengesetzten Forderungen auch von der Art und der Konzentration des zu vergarenden Substrates sowie von der jeweiligen Position innerhalb des Reaktors abhängt, wäre es wünschenswert, daß der Strömungszustand von außen, am besten getrennt für die einzelnen Reaktorabschnitte gesteuert werden kann.

Ein solcher Reaktor ist bekannt durch die EP 0 300 348 B1. Nachteilig ist bei den bekannten Anordnungen jedoch der relativ hohe konstruktive Aufwand. Ein Element der modular angeordneten Reaktoreinbauten besteht im einfachsten Fall aus einem Trichter, einem Rohr und an den Wänden angebrachten Abweiseelementen, die den Zugang von Gasblasen in die Sedimentationszonen vermeiden. Es wird eine Zirkulationsströmung erzeugt, die durch die Trennelemente geführt ist. Eine eindeutige Trennung der Strömung zwischen den Trennelementen wird somit nicht erzielt. Bei einer derartigen Ausbildung werden ferner in das Trennelement eindringende Gasblasen zum Teil mit der durch das Trennelement geführten Strömung mitgerissen und können daher in den Gassammelräumen nicht aufgefangen und als Biogas abgeführt werden. Zusätzlich ist daher in manchen Fällen ein weiteres Abweiseelement notwendig, um den direkten Zugang von aus dem Reaktorabschnitt eines tiefer liegenden Trennelementes aufsteigenden Gasblasen in den Rohrabschnitt des darüber liegenden Trennelements zu vermeiden. Dies ist besonders dann notwendig, wenn die Querschnittsfläche des Rohrabschnitts im Vergleich zur gesamten Reaktorquerschnittsfläche groß ist, wenn also beispielsweise die Querschnittsfläche des Rohrabschnitts 50% der Querschnittsfläche des gesamten Reaktors beträgt. In diesem Fall ist, betrachtet man die sich um ein Trennelement einstellende Zirkulationsströmung, der Strömungsguerschnitt der nach oben und der nach unten gerichteten Strömung in etwa gleich. Der Strömungswiderstand ist insgesamt gering. Bei einer solchen Dimensionierung der Trennelemente, die wegen der sich einstellenden günstigen Rührwirkung in manchen Fällen erwünscht ist, besteht nun die Gefahr, daß trotz einer direkten Versperrung des Blasenweges von unten zum oberen Trennelement Gasblasen durch die Ansaugwirkung der Zirkulationsströmung direkt in den oberen Rohrabschnitt gelangen und die Abscheideleistung des Trennelements damit verringert wird. Dieser ungünstige Zustand tritt ein in Fällen hoher örtlicher Biogasproduktion und gleichzeitig niederer Gasentnahme am Trennelement, mit der Folge einer starken Zirkulationsströmung. In diesem Fall ist die Steuerfähigkeit der Zirkulationsströmung eingeschränkt, da eine Gasentnahme nur den Anteil des Gases betrifft, der durch das Trennelement zurückgehalten wird.

In der DE-A- 37 23 851 ist ferner auch die Ausbildung eines Biogasreaktors mit trichterförmigen Trennelementen zur Behandlung von aktiver Biomasse beschrieben. Bei diesen Trennelementen ist die Spitze des Trichters nach unten gerichtet und weist eine Durchbrechung im Bereich der Trichterspitze auf. Der Trichter ist von einem zylinderförmigen Mantel derart umgeben, daß zwischen diesem Mantel und der Reaktorwand ein Ringkanal und zwischen dem Mantel und dem Trichter ein Gassammelraum ausgebildet ist, aus dem Gas über eine Gasabführleitung abgeführt wird. Bei diesem Trennelement besteht ebenfalls der Nachteil, daß durch die Ansaugwirkung der Zirkulationsströmung auf dem Weg von der Trichterspitze zum außenliegenden Ringkanal die aufsteigenden Gasblasen abgefangen und direkt in den äußeren Ringkanal geleitet werden. Auf diese Weise wird auch bei diesem Trennelement die Abscheideleistung im Fall einer starken Zirkulationsströmung verringert und die Steuerfähigkeit der Zirkulationsströmung eingeschränkt. Eine starke Drosselung der Zirkulationsströmung, etwa durch eine entsprechend kleine Dimensionierung der Durchbrechung an der Spitze, würde zwar die Abscheideleistung des Trennelementes sicherstellen aber die ebenfalls erwünschte, durch die Zirkulationsströmung bewirkte Durchmischung des Reaktorinhalts vermindern. Die angegebenen bekannten Reaktoren haben also bezüglich ihrer Steuerungsfähigkeit Nachteile, insbesondere in Fällen hoher Gasproduktionsleistung, die technisch jedoch besonders erwünscht ist.

Durch die WO 86/03 691 ist ferner ein turmförmiger, für die Biogasproduktion geeigneter Reaktor bekannt, der mehrere kaskadenförmig übereinander und in Strömungsrichtung hintereinander angeordnete Strömungszellen enthält. Die einzelnen Strömungszellen sind durch Strömungsleiteinrichtungen voneinander getrennt, die schräg nach oben gerichtet sind und durch die kein Gassammelraum gebildet werden kann. Dieser bekannte Reaktor erfüllt hinsichtlich einer durch die Biogasproduktion bewirkten guten Durchmischung des Reaktorinhalts die gestellten Forderungen. Durch diese Anordnung ist jedoch keine Ausbildung eines gasblasenarmen Sedimentationsraumes zur Sedimentation aktiver Biomasseschlammteilchen möglich, was aber für die Funktion eines Hochleistungs - Biogasreaktors Voraussetzung ist. Nicht möglich ist bei dieser bekannten Anordnung ferner eine Gasentnahme in tieferen Reaktorzonen und die erwünschte Steuerung der in den einzelnen Abschnitten ablaufenden Zirkulationsströmung. Hieran ändern auch nichts alternativ vorgesehene Einbauten von Leitrohren oder -blechen. Diese Leitrohre oder -bleche haben im übrigen eine weitere Aufgabe, nämlich als Heiz- oder Kühleinrichtungen zu dienen oder in den Reaktor Substrat einzutragen.

Die Aufgabe der Erfindung besteht darin, einen hochbauenden Biogasreaktor der eingangs genannten Art so auszubilden, daß insbesondere auch bei hohen Gasproduktionsraten in den einzelnen Reaktorsegmenten eine Strömung so eingestellt werden kann, daß ein ausreichender Rückhalt aktiver Biomasse möglich ist bei gleichzeitiger noch ausreichender guter Durchmischung.

Erfindungsgemäß erfolgt die Lösung der Aufgabe durch die kennzeichnenden Merkmale des Anspruchs 1. Vorteilhafte Ausgestaltungen der Erfindung werden in den abhängigen Ansprüchen beschrieben.

Mit dem erfindungsgemäßen Biogasreaktor ist es möglich, in allen Betriebszuständen, auch bei Gasproduktionsraten, die größer als acht Kubikmeter Biogas je Kubikmeter Reaktorvolumen und Tag sind, Gas aus tiefer liegenden Reaktorzonen abzuführen. Es wirkt stets ein Satz von zwei Trennelementen zusammen, die übereinander angeordnet und in der vertikalen Achse seitlich zueinander versetzt sind. Hierdurch bewirkt das an den Überströmkanten der Trennelemente überströmende Biogas oberhalb des jeweiligen Trennelements eine Zirkulationsströmung, die nicht in den Bereich der Gassammelräume eindringen kann. Die Ebene der Trennelemente wird somit von der Gärflüssigkeitströmung nicht durchdrungen. Durch die hierdurch mögliche Steuerung der jeweiligen Strömungszustände in den einzelnen Reaktorzonen kann in diesen ein optimaler Betrieb auch bei bezüglich Menge und Qualität wechselnden Abwasserzuläufen erzielt werden.

Die Erfindung wird nachstehend am Beispiel der in den Zeichnungen dargestellten Ausführungsbeispiele von Biogasreaktoren näher erläutert. Es zeigt:
- Fig. 1: einen Biogasreaktor in einer schematischen Seitenansicht,
- Fig. 2 und 3: den Biogasreaktor nach Fig. 1 mit vertikalen Leitflächen in der Seitenansicht im Ausschnitt,
- Fig. 4: einen Biogasreaktor mit kreissymmetrisch ausgebildeten Trennelementen in einer Seitenansicht im Ausschnitt,
- Fig. 5: eine im Durchmesser gegenüber dem Biogasreaktor nach Fig. 4 vergrößerte Ausbildung eines Biogasreaktors in der Seitenansicht im Ausschnitt,
- Fig. 6: einen rotationssymmetrischen Biogasreaktor mit zylinderförmigen Leitflächen in einer Seitenansicht im Schnitt,
- Fig. 7a bis 7c: ein Trennelement mit möglichen Ausbildungen der Überströmkante,
- Fig. 8 bis 10: weitere Ausbildungen eines Trennelementes in schematischen Seitenansichten,
- Fig. 11: eine Gasentnahmevorrichtung eines Trennelements in einer schematischen Seitenansicht.

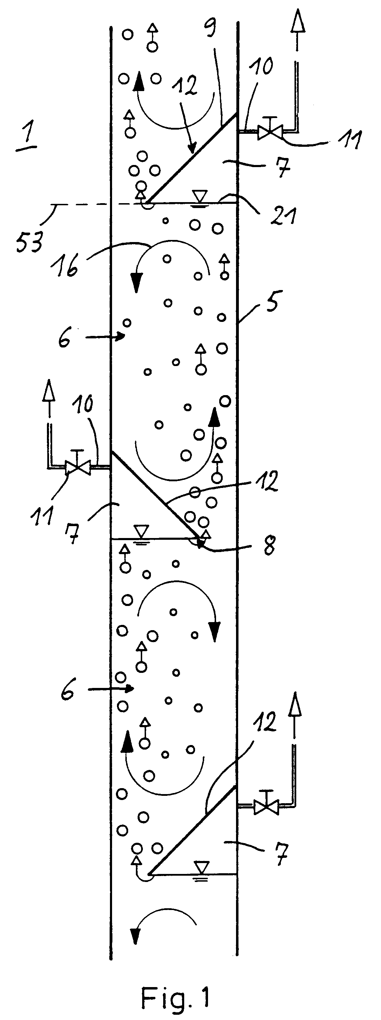

Fig. 1 zeigt einen Biogasreaktor 1, in dem in unterschiedlichen Reaktorhöhen durch Trennelemente 12 Reaktorzonen 6 ausgebildet sind. Die Trennelemente 12 sind als nach unten geneigte flächige Platten 9 ausgebildet, die mit der Reaktorwand des Reaktorgehäuses 5 jeweils einen Gassammelraum 7 bilden. In den Gassammelräumen 7 wird das von den jeweils darunter liegenden Reaktorzonen 6 aufsteigende Biogas aufgestaut. Entnimmt man über eine Leitung 10 mit Ventil 11 aus einem Gassammelraum 7 das gesamte aufgefangene Biogas, entsteht über diesem Trennelement 12 eine gasarme Zone, die die Sedimentation von Biogas fördert. Wird das Ventil 11 geschlossen, strömt Biogas an der äußeren Überströmkante 8 des Trennelements 12 über in den darüber liegenden Raum der folgenden Reaktorzone 6. Da das überströmende Biogas einseitig nur in der einen Raumhälfte aufsteigt, stellt sich eine die Durchmischung fördernde Zirkulationsströmung 16 ein. Die Geschwindigkeit der Zirkulationsströmung 16 erhöht sich mit steigendem Gasvolumenstrom. Durch die Entnahme von unterschiedlichen Gasstromvolumina durch Einstellung der Ventile 11 läßt sich die Zirkulationsströmung so einstellen, daß einerseits eine noch ausreichende Durchmischung erreicht und andererseits das Austragen von aktiver Biomasse zuverlässig vermieden wird. Dies wird dadurch erreicht, daß die in der jeweiligen Reaktorzone 6 erwünschte Zirkulationsströmung 16 der Gärflüssigkeit so definiert eingestellt wird, daß die Zirkulationsströmung keine der die Reaktorzone 6 begrenzenden horizontalen Trennelementebenen 53 durchdringt. Wie Experimente im halbtechnischen Bereich ergeben haben, variiert der optimal zu entnehmende Gasvolumenstrom in den unterschiedlichen Reaktorhöhen. So ist in tieferen Reaktorbereichen die Durchmischung zu fördern, während in den oberen Bereichen der Biomasserückhalt eher im Vordergrund steht.

Die Trennelemente 12 sind wechselnd auf jeweils gegenüberliegenden Seiten der Reaktorwand angeordnet. Auf diese Weise wird erreicht, daß das von einem darunter liegenden Trennelement 12 aufsteigende Biogas in optimaler Weise im Gassammelraum 7 des folgenden Trennelements 12 aufgefangen wird. Jeder Gassammelraum 7 ist nach unten durch einen Flüssigkeitsspiegel 21 als Grenzfläche zwischen Biogas und Gärsuspension begrenzt. Zusätzliche Abweiseeinrichtungen sind nicht notwendig. Eine ausreichende Gasabscheideleistung ist auch bei extrem hohen Gasproduktionsraten sowie bei vergleichsweise hoher Geschwindigkeit der Zirkulationsströmung 16 gewährleistet.

Die durch die Trennelemente geschaffenen Grenzflächen zwischen Biogas und Gärsuspension haben ferner den zusätzlichen Effekt, daß sich Agglomerate von Bakterien, die an von diesen produzierten Gasblasen haften, bis zu dieser Fläche aufsteigen. Diese kleinen Gasblasen werden nun von dem dort befindlichen großen Gasvolumen aufgenommen und die so vom Biogas befreiten bakteriellen Agglomerate können wieder in tiefere Zonen sedimentieren. Der Querschnitt des Biogasreaktors 1 kann beispielsweise eine kreisförmige oder rechteckige Form aufweisen.

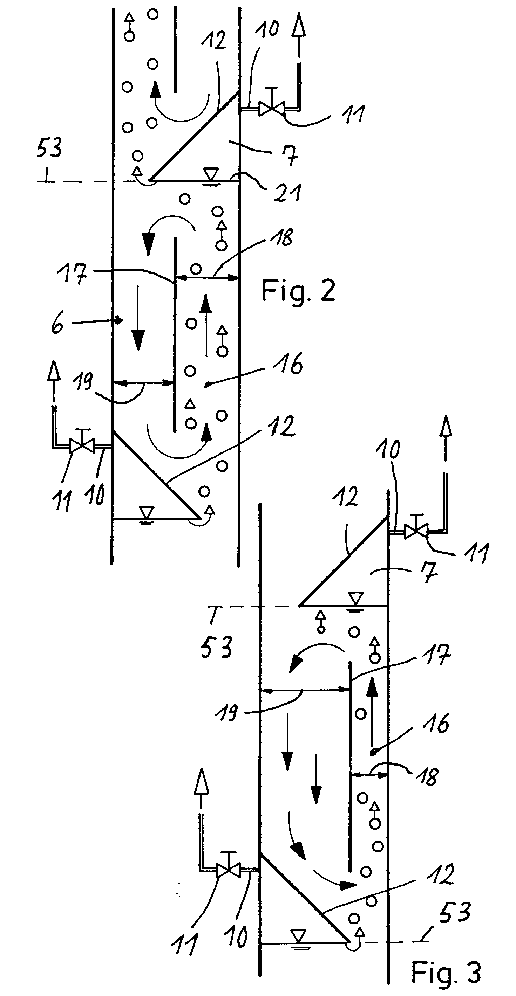

Zur Führung der Zirkulationsströmung 16 in den Reaktorzonen 6 können vertikale Leitflächen 17 in den Reaktorzonen 6 vorgesehen werden. Die Leitflächen 17 können mittig in den Reaktorzonen 6 angeordnet sein (Fig. 2). Es ist aber auch möglich, die Leitflächen 17 so anzuordnen, daß der Strömungsquerschnitt 18 der nach oben weisenden Strömung größer oder kleiner ist als der Strömungsquerschnitt 19 der nach unten weisenden Strömung. Ist der Querschnitt der nach oben weisenden Strömung kleiner, so wird aus Kontinuitätsgründen die Strömungsgeschwindigkeit in diesem Abschnitt erhöht. Auf diese Weise wird ein erhöhter Transport von Biomasse nach oben erreicht (Fig. 3). Bei Vergrößerung dieses Strömungsquerschnittes 18 tritt der umgekehrte Effekt auf.

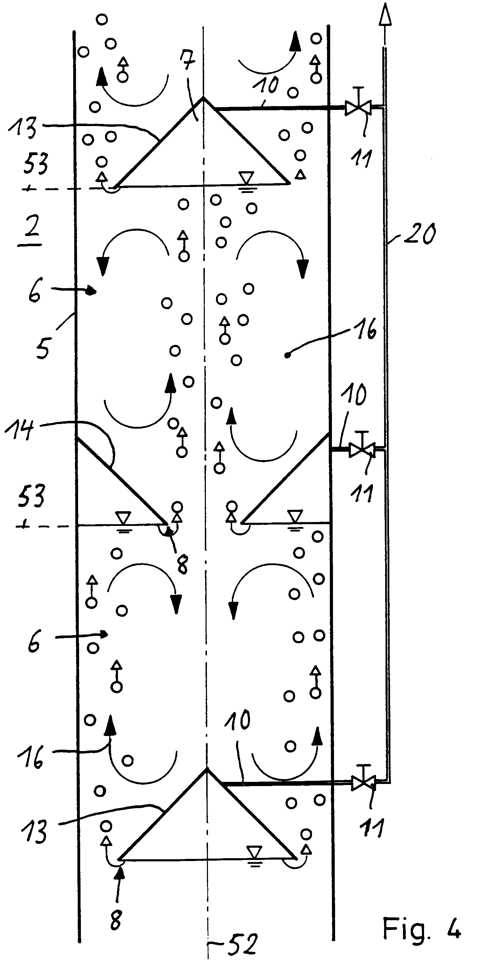

Fig. 4 zeigt eine Biogasreaktor 2, der kreissymmetrisch ausgebildet ist, wobei jede Reaktorzone 6 durch zwei Trennelemente 13, 14 definiert ist. Die Trennelemente 13 sind kegelförmig in der Mitte des Reaktorgehäuses 5 angeordnet und bilden somit einen mittigen Gassammelraum 7. Die Trennelemente 14 mit nach unten geneigter Plattenebene sind an der Reaktorwand des Reaktorgehäuses 5 angeordnet, so daß deren Überströmkanten 8 zur Mitte des Reaktorgehäuses 5 gerichtet sind. Die Trennelement 14 sind kreissymmetrisch ausgebildet. Die Gassammelräume 7 der Trennelemente 13, 14 sind jeweils mittels einer Leitung 10 mit Ventil 11 an eine zentrale Gasleitung 20 angeschlossen. In den Reaktorzonen 6 bildet sich koaxial zur Mittelachse 52 eine rotierende torusförmige Zirkulationsströmung 16 aus.

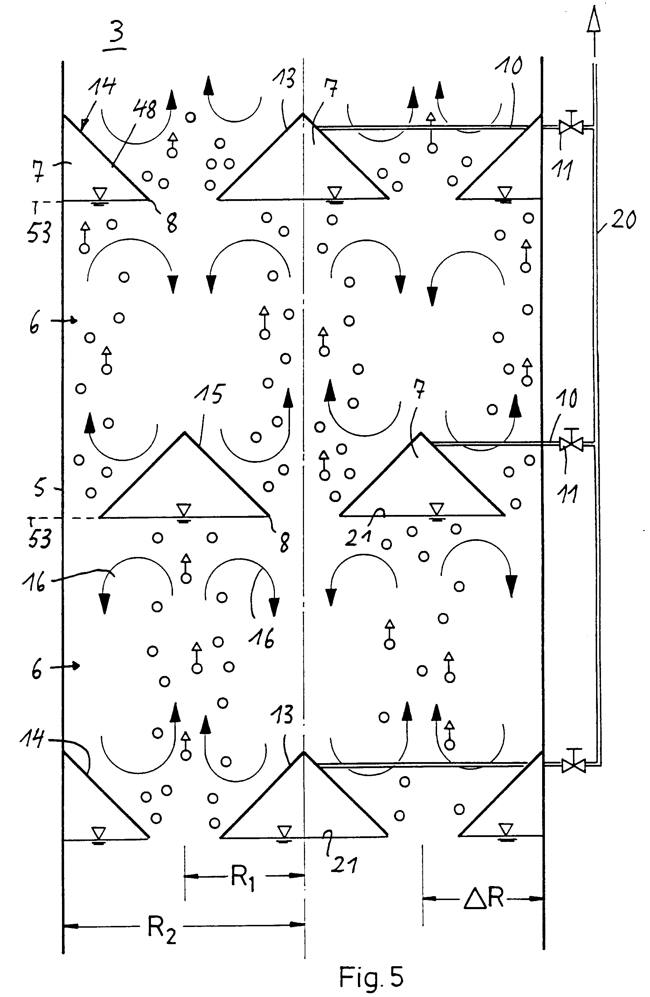

Der nutzbare Durchmesser des Biogasreaktors kann durch einen Umgruppierung der Trennelemente vergrößert werden, wobei die Querschnittsformen der Trennelemente 13, 14 erhalten bleiben. Fig. 5 zeigt hierzu schematisch einen Biogasreaktor 3, der wie der Biogasreaktor 2 kreissymmetrisch ausgebildet ist. Der Radius R1 ist lediglich um einen Betrag ΔR auf die Größe R2 vergrößert, wobei der Betrag ΔR dem Durchmesser des Biogasreaktors 1 entspricht. Dieser Biogasreaktor 3 weist drei unterschiedliche Trennelemente 13, 14, 15 auf. Das Trennelement 15 ist als kreisringförmiger Körper ausgebildet, der die Querschnittsform des Trennelements 13 aufweist. In den Reaktorzonen 6 zwischen den horizontalen Ebenen der Trennelemente 13, 14 und 15 bilden sich konzentrisch zueinander zwei torusförmige Zirkulationsströmungen 16 mit zueinander entgegengesetzter Strömungsrichtung aus. Die umlaufenden Trennelemente 14 sowie die Trennelemente 13, 15 ergeben im Fall einer großen Dimensionierung des Biogasreaktors einen großen Auftrieb. Die hierdurch entstehenden Kräfte müssen von der Reaktorwand aufgenommen werden. Die Kräfte können eventuell auch am Boden oder Deckel abgestützt werden.

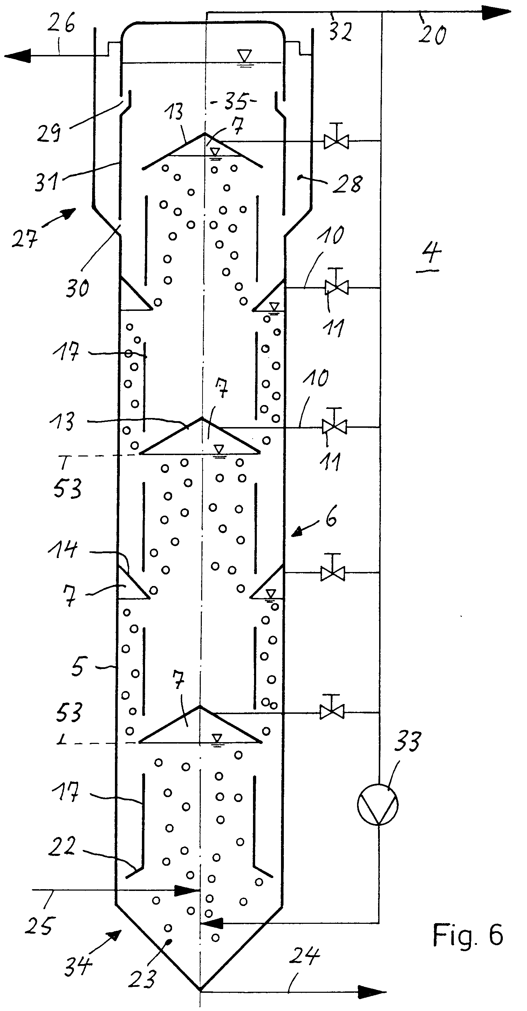

Fig. 6 zeigt einen weiteren rotationssymmetrischen Biogasreaktor 4 mit Trennelementen 13, 14, wie beim Biogasreaktor 2 nach Fig. 4, wobei jedoch in jeder Reaktorzone 6 eine zylinderförmige Leitfläche 17 angeordnet ist. Der untere Abschnitt 34 des Biogasreaktors 4 ist als Sedimentationsraum 23 ausgebildet. Hier anfallende biologisch nicht aktive Feststoffe können über die Abflußleitung 24 abgezogen werden. Eine Anschlußleitung 25 am unteren Abschnitt 34 des Biogasreaktors 4 dient zur Substratzufuhr. Am Reaktorkopf 27 ist eine Abflußleitung 26 für gereinigtes Abwasser und eine Gasleitung 32 angeschlossen, die mit einer zentralen Gasleitung 20 verbunden ist, durch die anfallendes Biogas zur weiteren Behandlung oder Verwertung abgeführt wird.

Um für das Anfahren des Biogasreaktors 4, wenn die Konzentration aktiver Biomasse noch niedrig und damit die produzierte Biogasmenge gering ist, eine ausreichende Durchmischung sicherzustellen, kann über einen Kompressor 33 zusätzlich Gas in untere Bereiche des Biogasreaktors 4 eingeführt werden. Es kann sich um sauerstoffreies Fremdgas wie z. B. Stickstoff oder um bereits produziertes Biogas handeln, das auf diese Weise im Kreis geführt wird. Es ist günstig, das Gas so einzuleiten, daß die aufsteigenden Gasblasen eine Einmischung des zugeführten Frischsubstrates bewirken. Um dies zu erleichtern, ist der untere Abschnitt der unteren zylinderförmigen Leitfläche 17 als sich konisch erweiternder Einlaufstutzen 22 ausgebildet. Der Reaktorkopf 27 weist im oberen Bereich eine Ringkammer 28 auf, die als zusätzliche für die Endabscheidung von Biomasse dienende Sedimentationszone dient. Das oberste trichterförmige Trennelement 13 wird durch Abzug von Biogas über die zugehörige Leitung 10 mit Ventil 11 so betrieben, daß kein Biogas in die darüber liegende Zone des Biogasreaktors 4 abgegeben wird. Der Sedimentationsraum der Ringkammer 28 ist mit dem Innenraum 35 des Reaktorkopfes 27 durch obere Öffnungen 29 und untere Öffnungen 30 in der Kammerinnenwand 31 verbunden. Da im Innenraum 35 des Reaktorkopfes 27 mit einem zwar geringen im Vergleich zur Ringkammer 28 jedoch leicht höheren Gasanteil in der Gärsuspension zu rechnen ist, wird sich über die Öffnungen 29, 30 eine langsame Zirkulationsströmung ausbilden, die in der Ringkammer 28 nach unten gerichtet ist und somit die Sedimentation unterstützt und für eine Rückführung sedimentierter Biomasse durch die Öffnung 30 in den Reaktorinnenraum sorgt. Wird die Öffnung 30 an einer Stelle angeordnet, an der auch im Reaktorinnenraum eine abwärts gerichtete Strömung auftritt, ergibt sich ein optimaler Rücktransport von Biomasse in tiefere Reaktorzonen 6.

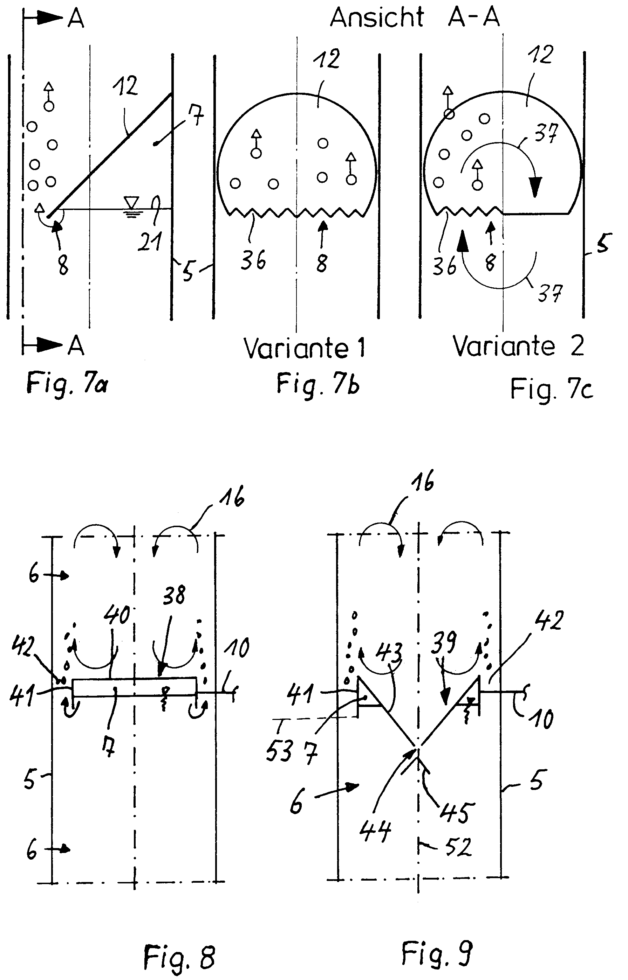

Die beispielhafte Ausbildung der Überströmkante 8 bei einem Trennelement 12 ist in den Fig. 7b und 7c als Ansicht A-A nach Fig. 7a dargestellt. Fig. 7a zeigt die Seitenansicht auf die Überströmkante 8 eines Trennelements 12 nach Fig. 1. Um ein schwallartiges Überströmen des aufgestauten Gases zu vermeiden, kann die Überströmkante 8 Kerben 36 enthalten (Fig. 7b). Werden nur an einem Teil der Überströmkante 8 Kerben 36 vorgesehen (Fig. 7c), bleibt die Gasentladung auf diesen Teil der Überströmkante 8 des Trennelements 12 beschränkt. Als Folge wird in einer senkrechten zur Überströmkante 8 parallelen Ebene eine Zirkulationsströmung 37 erzeugt, die den Stoffaustausch zwischen den über und unter dem Trennelement 12 befindlichen Räumen fördert. Auf diese Weise wird die gleichmäßige Substratverteilung innerhalb des Biogasreaktors verbessert.

Fig. 8 und 9 zeigen weitere Ausbildungen von Trennelementen 38, 39. Das Trennelement 38 besteht aus einer horizontalen konzentrisch im Reaktorgehäuse 5 angeordneten Platte 40, unter der durch einen randseitig nach unten umgekanteten Seitensteg 41 ein definierter Gassammelraum 7 ausgebildet ist. Das in diesem sich ansammelnde Biogas wird in bereits beschriebener Weise über eine Leitung 10 mit Ventil 11 abgeführt. Durch den Ringspalt 42 zwischen Seitensteg 41 und der Reaktorwand aufsteigendes Biogas bewirkt in der Reaktorzone 6 oberhalb des Trennelements 38 eine Zirkulationsströmung, die sich auf der Platte 40 des Trennelements 38 absetzende Mikroorganismen abspült. Bei intermittierendem Betrieb können sich die Organismen dagegen als aktiver Schlamm absetzen. Beim Einsatz dieses Trennelements 38 wird weniger Auftrieb erzeugt, so daß geringere Kräfte auf den Biogasreaktor einwirken und der für diesen erforderliche konstruktive Aufwand vermindert wird. Diese Trennelemente 38, 39 eignen sich daher besonders für größer dimensionierte Biogasreaktoren.

Das Trennelement 39 ist ebenfalls konzentrisch im Reaktorgehäuse 5 angeordnet und besteht aus einem mit der Spitze nach unten gerichteten trichterförmigen Grundkörper 43, der randseitig von einem ebenfalls nach unten gerichteten Seitensteg 41 umgeben ist. Durch diesen Seitensteg 41 wird ein umlaufender Gassammelraum 7 gebildet. In der Spitze des trichterförmigen Grundkörpers 43 ist eine Durchbrechung 44 ausgebildet, die so gestaltet ist, daß der oberhalb des Trennelementes 39 sedimentierte Schlamm durch die Durchbrechung 44 in die unter dem Trennelement 39 befindliche Reaktorzone 6 eintreten kann. Die Durchbrechung 44 wird hierbei möglichst klein dimensioniert jedoch andererseits ausreichend groß, so daß der Querschnitt durch den hindurchfließenden Schlamm nicht verstopft werden kann. Die Zirkulationsströmung 16, die sich bereits oberhalb des Trennelementes 39 schließt, wird von der Dimensionierung der Durchbrechung 44 nicht betroffen. Vor der Durchbrechung 44 befindet sich ein kegelförmiger Abweiser 45. Durch den Abweiser 45 wird verhindert, daß aufsteigende Gasblasen durch die Durchbrechung 44 in den Trichterinnenraum dringen und hierdurch in diesem eine Störung der Schlammsedimentation bewirken. Die Trennelemente 38 und 39 können entsprechend dem Trennelement 15 auch als kreisförmige Körper ausgebildet sein, der die Querschnittsform der Trennelemente 38 oder 39 aufweist.

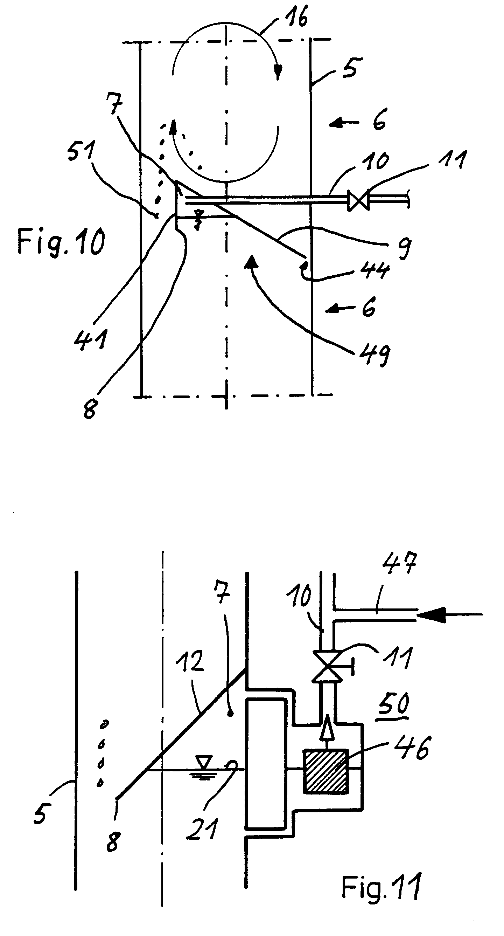

Fig. 10 zeigt eine weitere Ausbildung eines Trennelements 49. Funktional ist bei diesem im Ausschnitt dargestellten Biogasreaktor die Mittelachse 52 des Biogasreaktors nach Fig. 9 durch die Reaktorwand des Reaktorgehäuses 5 ersetzt. Das Trennelement 49 ist durch eine mit der Reaktorwand verbundene und zu dieser aufwärts gerichtete Platte 9 gebildet, an deren freiem Endabschnitt ein nach unten gerichteter Seitensteg 41 angeordnet ist. Zwischen diesem und der Platte 9 ist ein Gassammelraum 7 ausgebildet, der über eine Leitung 10 mit Ventil 11 mit einer nicht näher dargestellten zentralen Gasleitung verbunden ist. Zwischen dem Seitensteg 41 und der Reaktorwand ist ein Überströmkanal 51 ausgebildet, durch den über die Überströmkante 8 abströmendes Biogas in die nächste obere Reaktorzone 6 strömen kann. Am tiefsten Punkt der Platte 9 ist in dieser ein Durchbrechung 44 vorhanden. Durch diese Durchbrechung 44 kann fließfähiger Schlamm von dem Schlammraum oberhalb der Platte 9 in die unter dieser befindliche Reaktorzone 6 zurückfließen.

In Fig. 11 ist eine Ausführungsform einer einem Trennelement 12 zugeordneten Gasentnahmevorrichtung 50 dargestellt. Um das Ventil 11 vor einer möglichen Überflutung durch Reaktorinhalt zu schützen, ist die Gasentnahme oberhalb eines bestimmten Flüssigkeitsstandes durch ein Schwimmerventil 46 begrenzt. Um die Funktion des Schwimmerventils 46 und/oder des Ventils 11 zur Gasentnahme bei einer möglichen Verschmutzung zu erhalten, kann eine entgegen der Gasströmungsrichtung gerichtete Flüssigkeitspülung vorgesehen werden. Hierzu ist an die das Ventil 11 aufweisende Leitung 10 eine Spülleitung 47 angeschlossen. Eine Verschmutzung ist insbesondere beim Auftreten von Schaum im Gassammelraum 7 zu befürchten. Als Spülflüssigkeit kann entweder frisches Leitungswasser oder unvergorenes Substrat oder bereits behandeltes Abwasser eingesetzt werden.

## Patentansprüche

1. Biogasreaktor, dessen Reaktorgehäuse (5) ganz oder teilweise mit aktiver Biomasse gefüllt ist, mit einem Trennelement (12, 13, 14, 15, 38, 39, 49) oder mehreren säulenartig übereinander angeordneten Trennelementen (12, 13, 14, 15, 38, 39, 49) mit jeweils einer Überströmkante (8) für aufsteigendes Gas, wobei die durch die Trennelemente (12, 13, 14, 15, 38, 39, 49) gebildeten Reaktorabschnitte durch die Trennelemente (12, 13, 14, 15, 38, 39, 49) jeweils einen Gassammelraum (7) zum Sammeln der aus der Gärsuspension aufsteigenden Gase und einen gasblasenarmen Sedimentationsraum zur Sedimentation aktiver Biomasse-Schlammteilchen aufweisen und in jeden Gassammelraum (7) eine Leitung (10) mit einem als Ventil (11) ausgebildeten Drosselorgan zur Gasabführung und zur Einstellung des in die oberhalb des jeweiligen Trennelements (12, 13, 14, 15, 38, 39, 49) befindlichen Reaktorzone (6) überströmenden Biogasvolumens geführt ist, dadurch gekennzeichnet, daß jedes Trennelement (12, 13, 14, 15, 38, 39, 49) als nach oben geschlossener Gassammelraum (7) ausgebildet ist, daß die Trennelemente (12, 13, 14, 15, 38, 39, 49) der einen horizontalen Trennelementebene (53) in der vertikalen Achse seitlich jeweils versetzt zu den Trennelementen (12, 13, 14, 15, 38, 39, 49) der jeweils unmittelbar darüber und darunter befindlichen horizontalen Trennelementebene (53) angeordnet sind, und daß die oberhalb und unterhalb eines jeden Trennelements (12, 13, 14, 15, 38, 39, 49) befindlichen Reaktorzonen (6) nur mittels einer Öffnung miteinander verbunden sind, die jeweils an der dem Gassammelraum (7) zugeordneten Überströmkante (8) des jeweiligen Trennelements (12, 13, 14, 15, 38, 39, 49) ausgebildet ist und durch die das über die Überströmkante (8) aus dem Gassammelraum (7) austretende Biogas in die oberhalb des Trennelements (12, 13, 14, 15, 38, 39, 49) befindliche Reaktorzone (6) einströmt.

2. Biogasreaktor nach Anspruch 1, dadurch gekennzeichnet, daß das Trennelement (12) als einseitig an der Reaktorwand angeordnete und zu dieser schiefwinklig nach unten geneigte Platte (9) ausgebildet ist.

3. Biogasreaktor nach Anspruch 1, dadurch gekennzeichnet, daß das Trennelement (13) als trichterförmiger Körper mit nach oben gerichteter geschlossener Spitze ausgebildet ist.

4. Biogasreaktor nach Anspruch 1, dadurch gekennzeichnet, daß das Trennelement (14) als einseitig an der Reaktorwand angeordneter und zu dieser schiefwinklig nach unten geneigter Kreisring (48) ausgebildet ist.

5. Biogasreaktor nach Anspruch 1, dadurch gekennzeichnet, daß das Trennelement (15) als kreisringförmiger Körper mit trichterförmigem Querschnitt ausgebildet ist, dessen verschlossene Spitze nach oben gerichtet ist.

6. Biogasreaktor nach Anspruch 1, dadurch gekennzeichnet, daß das Trennelement (38) aus einer horizontalen Platte (40) besteht, an der randseitig ein umlaufender nach unten gerichteter Seitensteg (41) angeformt ist.

7. Biogasreaktor nach Anspruch 1, dadurch gekennzeichnet, daß das Trennelement (39) aus einem mit der Spitze nach unten gerichteten trichterförmigen Grundkörper (43) besteht, an dem randseitig ein umlaufender nach unten gerichteter Seitensteg (41) angeformt und in dessen Spitze eine Durchbrechung (44) zum Austritt sedimentierten Schlamms ausgebildet ist.

8. Biogasreaktor nach Anspruch 6 und 7, dadurch gekennzeichnet, daß die horizontale Platte (40) und der trichterförmige Grundkörper (43) in einer horizontalen Ebene kreisringförmig ausgebildet sind.

9. Biogasreaktor nach Anspruch 7, dadurch gekennzeichnet, daß im Abstand von der Spitze des trichterförmigen Grundkörpers (43) ein Abweiser (45) angeordnet ist.

10. Biogasreaktor nach Anspruch 8, dadurch gekennzeichnet, daß der Abweiser (45) im Querschnitt kegelförmig mit nach oben gerichteter Spitze ausgebildet ist.

11. Biogasreaktor nach Anspruch 8 bis 10, dadurch gekennzeichnet, daß der Abweiser (45) in einer horizontalen Ebene kreisringförmig ausgebildet ist.

12. Biogasreaktor nach Anspruch 1, dadurch gekennzeichnet, daß das Trennelement (49) aus einer einseitig an der Reaktorwand angeordneten und zu dieser schiefwinklig nach oben geneigten Platte (9) besteht, an deren freiem Endabschnitt ein nach unten gerichteter Seitensteg (41) und an deren tiefster Stelle an der Reaktorwand eine Durchbrechung (44) ausgebildet ist.

13. Biogasreaktor nach Anspruch 1 bis 12, dadurch gekennzeichnet, daß in dem Reaktorgehäuse (5) zur Ausbildung vertikal übereinander befindlicher Reaktorzonen (6) eine Kombination von Trennelementen (12) und/oder (13) und/oder (14) und/oder (15) und/oder (38) und/oder (39) und/oder (49) angeordnet ist, wobei die in einer Trennelementebene (53) angeordneten Trennelemente (12, 13, 14, 15, 38, 39, 49) im Bereich zugewandter Abschnitte der Überströmkanten (8) eine gemeinsame Öffnung zum Überströmen des aus dem jeweiligen Gassammelraums (7) austretenden Biogases durch die Trennelementebene (53) in die oberhalb dieser Trennelemente (12, 13, 14, 15, 38, 39, 49) befindliche Reaktorzone (6) aufweisen.

14. Biogasreaktor nach Anspruch 1 bis 13, dadurch gekennzeichnet, daß der Reaktorkopf (27) von einer als Sedimentationsraum dienenden Ringkammer (28) umgeben ist, die über in der Kammerinnenwand (31) oberhalb und unterhalb des obersten Trennelements ausgebildete Öffnungen (29, 30) mit dem oberhalb des obersten Trennelements befindlichen Innenraum (35) des Reaktorkopfes (27) und der unterhalb des obersten Trennelements befindlichen Reaktorzone (6) verbunden ist.

15. Biogasreaktor nach Anspruch 1 bis 14, dadurch gekennzeichnet, daß in den als Sedimentationsraum (23) ausgebildeten unteren Abschnitt (34) des Reaktorgehäuses (5) dosiert ein Gas zur Durchmischung der Biomasse einleitbar ist.

16. Biogasreaktor nach Anspruch 1 bis 15, dadurch gekennzeichnet, daß in mindestens einer Reaktorzone (6) eine vertikale Leitfläche (17) für die Zirkulationsströmung (16) angeordnet ist.

17. Biogasreaktor nach Anspruch 16, dadurch gekennzeichnet, daß die mindestens eine vertikale Leitfläche (17) zylinderförmig ausgebildet ist.

18. Biogasreaktor nach Anspruch 17, dadurch gekennzeichnet, daß an der in der untersten Reaktorzone (6) angeordneten zylinderförmigen Leitfläche (17) ein Einlaufstutzen (22) ausgebildet ist.

19. Biogasreaktor nach Anspruch 1, bei dem jeder Gassammelraum (7) mit einer Gasentnahmevorrichtung (50) mit einem der Leitung (10) zur Gasentnahme zugeordneten Schwimmerventil (46) in Verbindung steht, dadurch gekennzeichnet, daß an die Leitung (10) eine Spülleitung (47) angeschlossen ist, über die Spülflüssigkeit dem Ventil (11) und dem Schwimmerventil (46) zuführbar ist.

## Claims

1. Biogas reactor, whose reactor housing (5) is fully or partially filled with a dividing element (12, 13, 14, 15, 38, 39, 49) or several column-type elements (12, 13, 14, 15, 38, 39, 49) arranged one above the other, having respectively a face (8) for directing the flow of rising gas, whereby the reactor sections formed between the dividing elements (12, 13, 14, 15, 38, 39, 49) form by means of the dividing elements (12, 13, 14, 15, 38, 39, 49) respective gas collection chambers (7) for collecting the gases rising from the ferment suspension and a sedimentation chamber (7) that is low in gas bubbles for the sedimentation of active biomass sludge particles and, feeding into each gas collection chamber (7), a pipe (10) with a throttle element in the form of a valve (11) for evacuating the gas and for adjusting the volume of biogas flowing into the reactor zone (6) above the respective dividing element (12, 13, 14, 15, 38, 39, 49), characterised in that each dividing element (12, 13, 14, 15, 38, 39, 49) is constructed as a gas collection chamber (7) with a closed upper part, that the dividing elements (12, 13, 14, 15, 38, 39, 49) within the one horizontal plane (53) of dividing elements are respectively laterally offset on the vertical axis from the dividing elements (12, 13, 14, 15, 38, 39, 49) of the respective horizontal plane (53) directly thereover or thereunder and that the reactor zones (6) located above and below each of the dividing elements (12, 13, 14, 15, 38, 39, 49) are connected to each other by one opening only which is arranged respectively on the respective faces (8) of each dividing element that directs the flow to the respective gas collection chamber (7) and whereby the biogas flows out of the gas collection chamber (7) over the flow-directing face (8) into the reactor zone (6) located above the dividing element (12, 13, 14, 15, 38, 39, 49).

2. Biogas reactor as claimed in claim 1, characterised in that the dividing element (12) is constructed as a plate (9) one side of which is arranged on the side of the reactor wall and downwardly inclined at a sharp angle thereto.

3. Biogas reactor as claimed in claim 1, characterised in that the dividing element (13) is constructed as a funnel-shaped body with an upwardly pointing, closed apex.

4. Biogas reactor as claimed in claim 1, characterised in that the dividing element (14) is constructed as an annular ring (48) one side of which is arranged on the reactor wall and downwardly inclined at a sharp angle thereto.

5. Biogas reactor as claimed in claim 1, characterised in that the dividing element (15) is constructed as an annular-shaped body with a funnel-shaped cross-section, having an upwardly pointing, closed apex.

6. Biogas reactor as claimed in claim 1, characterised in that the dividing element (38) consists of a horizontal plate, around the peripheral edge of which runs a downwardly pointing edge-piece (41).

7. Biogas reactor as claimed claim 1, characterised in that the dividing element (39) consists of a funnel-shaped main body (43) with a downwardly pointing apex, around the peripheral edge of which runs a downwardly pointing edge-piece (41), an orifice (44) being arranged in the apex thereof to permit evacuation of the sedimented sludge.

8. Biogas reactor as claimed in claims 6 and 7, characterised in that the horizontal plate (40) and the funnel-shaped main body (43) are constructed in an annular shape in a horizontal plane.

9. Biogas reactor as claimed in claim 7, characterised in that a blocking element (45) is arranged at a distance from the apex of the funnel-shaped main body (43).

10. Biogas reactor as claimed in claim 8, characterised in that the blocking element (45) is wedge-shaped in cross-section with an upwardly pointing apex.

11. Biogas reactor as claimed in claims 8 to 10, characterised in that the blocking element (45) is constructed in an annular shape in a horizontal plane.

12. Biogas reactor as claimed in claim 1, characterised in that the dividing element (49) consists of a plate (9) one side of which is arranged against the reactor wall and upwardly inclined at a sharp angle thereto, having at the free end portion a downwardly pointing edge-piece (41), an orifice (44) being arranged at the deepest point on the reactor wall.

13. Biogas reactor as claimed in claims 1 to 12, characterised in that for the purpose of forming reactor zones (6) arranged vertically one above the other in the reactor housing (5), a combination of dividing elements (12) and/or (13) and/or (14) and/or (15) and/or (38) and/or (39) and/or (49) is provided, whereby the dividing elements (12, 13, 14, 15, 38, 39, 49) arranged in a plane (53) of dividing elements have, in the area of the portions facing the flow-directing faces (8), a common orifice to allow the biogas to flow from the respective gas collection chambers (7) through the plane of dividing elements (53) into the reactor zones (6) above these dividing elements (12, 13, 14, 15, 38, 39, 49).

14. Biogas reactor as claimed in claims 1 to 13, characterised in that the reactor head (27) is surrounded by an annular chamber (28) serving as a sedimentation chamber, which is connected via orifices (29, 30) arranged in the inner wall of the chamber (31) above and below the uppermost dividing element with the inner chamber (35) of the reactor head (27) above the uppermost dividing element and the reactor zone 6 below the uppermost dividing element.

15. Biogas reactor as claimed in claims 1 to 14, characterised in that a gas is measured and fed into the lower portion (34) of the reactor housing (5) arranged as a sedimentation chamber (23) for mixing with the biomass.

16. Biogas reactor as claimed in claims 1 to 15, characterised in that a vertical guide surface (17) is arranged in at least one reactor zone (6) to direct the circuit flow (16).

17. Biogas reactor as claimed in claim 16, characterised in that the at least one vertical guide surface (17) is cylindrical in shape.

18. Biogas reactor as claimed in claim 17, characterised in that an inlet connecting piece (22) is arranged on the cylindrical-shaped guide surface (17) arranged in the lowest reactor zone (6).

19. Biogas reactor as claimed in claim 1, in which each gas collection chamber (7) is connected to a gas discharge device (50) with a float valve (46) arranged on the gas discharge pipe (10), characterised in that a scavenging pipe is connected to pipe (10) through which scavenging fluid can be fed via the valve (11) and float valve (46).

## Revendications

1. Réacteur de biogaz dont l'enceinte de réacteur (5) est complétement ou partiellement remplie de biomasse active, avec un élément de séparation (12, 13, 14, 15, 38, 39, 49) ou avec plusieurs éléments de séparation (12, 13, 14, 15, 38, 39, 49) du genre colonne les uns au-dessus des autres chacun avec un rebord de débordement (8) pour le gaz montant, dans lequel chacune des sections de réacteur formées par les éléments de séparation (12, 13, 14, 15, 38, 39, 49) constituent, par l'intermédiaire des éléments de séparation (12, 13, 14, 15, 38, 39, 49), une enceinte de récupération de gaz (7) afin de récupérer les gaz montant de la suspension de ferments et une enceinte de sédimentation pauvre en bulles de gaz pour la sédimentation de particules actives de boue de biomasse et, dans chaque enceinte de récupération de gaz (7), est prévue une canalisation (10) avec un organe d'étranglement sous forme de vanne (11) pour l'échappement des gaz et le réglage du volume de biogaz se dégageant dans la zone de réacteur (6) située dans la moitié supérieure de chaque élément de séparation respectif (12, 13, 14, 15, 38, 39, 49), caractérisé en ce que chaque élément de séparation (12, 13, 14, 15, 38, 39, 49) est constitué en tant qu'enceinte de récupération de gaz (7) fermée vers le haut, en ce que les éléments de séparation (12, 13, 14, 15, 38, 39, 49) sont disposés selon un plan horizontal (53) d'élément de séparation, qui sont décalés chacun latéralement autour de l'axe vertical par rapport aux éléments de séparation (12, 13, 14, 15, 38, 39, 49) de chaque plan horizontal (53) d'élément de séparation se trouvant directement au-dessus et au-dessous, et que les zones de réacteur (6) se trouvant au-dessus et au-dessous de chaque élément de séparation (12, 13, 14, 15, 38, 39, 49) ne sont reliées entre elles que par une ouverture, qui est formée, à chaque fois, par le rebord de débordement associé à l'enceinte de récupération de gaz (7) de l'élément de séparation (12, 13, 14, 15, 38, 39, 49) et au travers de laquelle le biogaz sortant par le rebord de débordement (8) de l'enceinte de récupération de gaz (7) s'écoule dans la zone de réacteur (6) située au-dessus de l'élément de séparation (12, 13, 14, 15, 38, 39, 49).

2. Réacteur de biogaz selon la revendication 1, caractérisé en ce que l'élément de séparation (12) est prévu comme ayant un côté contre la paroi de réacteur et formant un angle oblique avec la plaque (9) inclinée vers le bas.

3. Réacteur de biogaz selon la revendication 1, caractérisé en ce que l'élément de séparation (13) est un corps en forme d'entonnoir avec la pointe ferméé dirigée vers le haut.

4. Réacteur de biogaz selon la revendication 1, caractérisé en ce que l'élément de séparation (14) est prévu sous forme d'anneau circulaire ayant un côté contre la paroi de réacteur et formant un angle oblique avec celle-ci et incliné vers le bas.

5. Réacteur de biogaz selon la revendication 1, caractérisé en ce que l'élément de séparation (15) est prévu sous forme de corps en boucle circulaire à section en forme d'entonnoir, dont la pointe fermée est dirigée vers le haut.

6. Réacteur de biogaz selon la revendication 1, caractérisé en ce que l'élément de séparation (38) est constitué d'une plaque horizontale, sur les bords de laquelle est formé un patte latérale circulaire dirigée vers le bas.

7. Réacteur de biogaz selon la revendication 1, caractérisé en ce que l'élément de séparation (39) est constitué d'un corps de base en forme d'entonnoir avec la pointe dirigée vers le bas, autour duquel est formé un profil latéral enveloppant dirigé vers le bas, dont la pointe comporte une ouverture (44) destinée à la sortie de boue sédimentée.

8. Réacteur de biogaz selon les revendications 6 et 7, caractérisé en ce que la plaque horizontale (40) et le corps de base en forme d'entonnoir (43) sont prévus sous forme d'anneau circulaire dans un plan horizontal.

9. Réacteur de biogaz selon la revendication 7, caractérisé en ce qu'un déflecteur (45) est placé à une certaine distance de la pointe du corps de base en forme d'entonnoir (43).

10. Réacteur de biogaz selon la revendication 8, caractérisé en ce que le déflecteur (45) a une section en forme de cône avec la pointe dirigée vers le haut.

11. Réacteur de biogaz selon les revendications 8 à 10, caractérisé en ce que le déflecteur (45) est fourni sous forme d'anneau circulaire dans un plan horizontal.

12. Réacteur de biogaz selon la revendication 1, caractérisé en ce que l'élément de séparation (49) est constitué d'une plaque (9) fixée d'un côté contre la paroi du réacteur, inclinée d'un angle faible vers le haut par rapport à celle-ci, à l'extrémité libre de laquelle on a réalisé un rebord latéral (41), et à la position la plus basse duquel on a réalisé une ouverture (44).

13. Réacteur de biogaz selon les revendications 1 à 12, caractérisé en ce qu'une combinaison d'éléments de séparation (12) et/ou (13) et/ou (14) et/ou (15) et/ou (38) et/ou (39) et/ou (49) est disposée dans l'enceinte de réacteur (5) pour former des zones de réacteur verticales (6) se trouvant les unes au-dessus des autres, dans lequel les éléments de séparation (12, 13, 14, 15, 38, 39, 49) disposés dans un plan d'élément de séparation (53), présentent dans la région faisant face aux sections de rebords de débordement (8) une ouverture commune pour l'écoulement du biogaz sortant de l'enceinte de récupération de gaz (7) au travers du plan d'élément de séparation (53) dans les zones de réacteur (6) situées au-dessus de ces éléments de séparation (12, 13, 14, 15, 38, 39, 49).

14. Réacteur de biogaz selon les revendications 1 à 13, caractérisé en ce que le sommet (27) du réacteur est entouré d'une enceinte annulaire (28) servant à enceinte de sédimentation, qui est reliée à l'enceinte interne (35) se trouvant au-dessus de l'élément de séparation le plus élevé et de la zone de réacteur (6) se trouvant au-dessous de l'élément de séparation le plus élevé, au moyen des ouvertures (29,30) formées dans la paroi de l'enceinte interne (31) et se trouvant au-dessus et au-dessous de l'élément de séparation le plus élevé.

15. Réacteur de biogaz selon les revendications 1 à 14, caractérisé en ce que l'on peut introduire un gaz en quantité dosée pour être mélangé à la biomasse dans la section inférieure (34) de l'enceinte du réacteur (5), constituée en tant qu'enceinte de sédimentation (23).

16. Réacteur de biogaz selon les revendications 1 à 15, caractérisé en ce qu'il comporte, dans au moins une zone de réacteur (6), d'une surface de canalisation verticale (17) pour l'écoulement circulant (16).

17. Réacteur de biogaz selon la revendication 16, caractérisé en ce qu'il comporte au moins une surface de canalisation verticale (17) de forme cylindrique.

18. Réacteur de biogaz selon la revendication 17, caractérisé en ce qu'il est fourni un rétrécissement (22) de l'écoulement à la surface de canalisation de forme cylindrique (17) se trouvant dans la zone de réacteur (6) la plus basse.

19. Réacteur de biogaz selon la revendication 1, dans lequel chaque enceinte de récupération de gaz (7) est en liaison avec un dispositif d'évacuation du gaz (50), avec une vanne à flotteur (46) fixée à la canalisation (10) d'évacuation de gaz, caractérisé en ce qu'une canalisation de rinçage (47) est raccordée à la canalisation (10), par laquelle du fluide de rinçage peut être conduit à la vanne (11) et à la vanne à flotteur (46).
